# EUROPÄISCHE PATENTSCHRIFT

(11) **EP 1 519 686 B1**
(45) Veröffentlichungstag und Bekanntmachung des Hinweises auf die Patenterteilung: **11.01.2006**
(21) Anmeldenummer: 03762572.0
(22) Anmeldetag: 01.07.2003
(51) Int. Cl.: A61B 17/02

(54) **BÄNDERSPANNVORRICHTUNG MIT VERSCHIEBLICHEN PRATZEN**
LIGAMENT TENSING DEVICE WITH DISPLACEABLE LUG
DISPOSITIF DE TENSION DE LIGAMENTS A GRIFFES MOBILES

(30) Priorität: 05.07.2002 DE 10230375
(43) Veröffentlichungstag der Anmeldung: 06.04.2005
(73) Patentinhaber: Mathys Medizinaltechnik AG, 2544 Bettlach (CH)
(72) Erfinder: SUPPER, Walter, CH-2544 Bettlach (CH); FANKHAUSER, Christoph, CH-4500 Solothurn (CH); GRUNDER, Beat, CH-3076 Worb (CH); DELFOSSE, Daniel, CH-3018 Bern (CH); WEHRLI, Ulrich, CH-1789 Lugnorre (CH)
(74) Vertreter: Körfer, Thomas
(86) Internationale Anmeldenummer: PCT/EP2003/007012
(87) Internationale Veröffentlichungsnummer: WO 2004/004576

(56) Entgegenhaltungen:
- WO-A-00/78225
- US-A- 5 213 112
- US-A- 5 468 244

## Beschreibung

Die Erfindung betrifft eine Bänderspannvorrichtung für nichtkugelige Gelenke des menschlichen oder tierischen Körpers.

Aus der WO 00/78225 A1 ist eine Bänderspannvorrichtung für nicht-kugelige Gelenke bekannt. Die darin beschriebene Vorrichtung zum Spannen von Bändern an nicht-kugeligen Gelenken am menschlichen oder tierischen Körper umfaßt einen prismatischen, zylindrischen oder plattenförmigen Grundkörper mit einer rechten Pratze und einer linken Pratze, welche erste Auflageflächen in einer Ebene aufweisen und damit parallel auf die gelenkseitige Oberfläche eines ersten an ein nicht-kugeliges Gelenk angrenzenden Knochens zur Anlage bringbar sind. Die Bänderspannvorrichtung hat einen rechten Handgriff und einen linken Handgriff, einen rechten Spannhebel und einen linken Spannhebel mit zweiten Auflageflächen, welche parallel zu den ersten Auflageflächen angeordnet sind, wobei zwischen den jeweiligen Auflageflächen des rechten Spannhebels und der rechten Pratze eine Spannweite Y und zwischen den jeweiligen Auflageflächen des linken Spannhebels und der linken Pratze dieselbe oder eine andere Spannweite X einstellbar ist. Die zweiten Auflageflächen sind auf die gelenkseitige Oberfläche eines zweiten an das Gelenk angrenzenden Knochens zur Anlage bringbar. Weiterhin umfaßt die Vorrichtung einen rechten Bedienungshebel und einen linken Bedienungshebel, welche gleichzeitig mit dem Halten der Vorrichtung mit je einer Hand am entsprechenden Handgriff einzeln mit der jeweils selben Hand betätigbar sind und eine rechte Parallelverschiebevorrichtung und eine linke Parallelverschiebevorrichtung, welche je durch den entsprechenden Bedienungshebel antreibbar sind und so mit je einem Spannhebel verbunden sind, daß bei einer Bewegung der Bedienungshebel die Spannweiten X bzw. Y unabhängig voneinander einstellbar sind. Die Parallelverschiebevorrichtungen sind als Viergelenk-Hebelgetriebe ausgebildet.

Nachteilig an der aus der WO 00/78225 A1 bekannten Bänderspannvorrichtung ist insbesondere, daß das Gelenk nach der Anspannung der Ligamente (Bänder) in sich verspannt ist. Die Spannungen führen zu einer hohen Reibungskraft zwischen der Bänderspannvorrichtung und dem auf dieser aufliegenden Knochen. Dadurch kann es im Operationsverlauf sowohl zu Schädigungen, des anliegenden Knochengewebes durch die Reibung als auch zu plötzlichen Verschiebungen der Bänderspannvorrichtung und/oder des Knochens durch die Spannung der Ligamente kommen, welche nicht kontrollierbar sind und daher den Operationsverlauf empfindlich stören.

Der Erfindung liegt demnach die Aufgabe zugrunde, eine Bänderspannvorrichtung zu schaffen, die es ermöglicht, die Kapsel-Bandstrukturen eines prothetisch zu versorgenden Gelenkes mit einer parallelen Spreizbewegung anzuspannen und zugleich Querspannungen mit hohen Reibkräften zu vermeiden.

Die Aufgabe wird durch die Merkmale des Anspruchs 1 gelöst.

Weitere vorteilhafte Ausgestaltungen der Erfindung sind in den Unteransprüchen gekennzeichnet.

Vorteilhafterweise ist die zweite Pratze dabei zweiteilig ausgeführt, wobei der dem Knochen zugewandte Teil gegenüber dem mit der Bänderspannvorrichtung verbundenen Teil der Pratze verschiebbar ist.

Weiterhin ist von Vorteil, daß der bewegliche Teil der Pratze mittels eines Dorns in einer entsprechenden Führung des mit der Bänderspannvorrichtung verbundenen Teils der Pratze geführt ist.

Eine Arretierungsvorrichtung, welche beispielsweise als Kipp- oder Wipphebel ausgebildet sein kann, sorgt in vorteilhafter Weise beim Einführen der Bänderspannvorrichtung für eine sichere Arretierung und nach dem Aufspreizen für eine einfache Entriegelung des beweglichen Teils der Pratze.

Besonders vorteilhaft ist dabei die unabhängige Verstellbarkeit in cranio-caudaler sowie anterio-posteriorer Richtung. Die Verschiebung in cranio-caudaler Richtung kann dabei beispielsweise mittels einer quantifizierbaren Parallelverschiebevorrichtung erfolgen.

Weiterhin ist von Vorteil, daß beliebige rotatorische und translatorische Freiheitsgrade realisierbar sind, um den individuellen anatomischen Randbedingungen eines beliebigen (Knie-)Gelenks Rechnung tragen zu können.

Die Erfindung wird im folgenden anhand schematischer Darstellungen in verschiedenen Perspektiven näher erläutert.

Es zeigen:
- Fig. 1: eine schematische, perspektivische Ansicht eines Ausführungsbeispiels einer erfindungsgemäßen Bänderspannvorrichtung, und
- Fig. 2: eine schematische Aufsicht auf das in Fig. 1 dargestellte Ausführungsbeispiel einer erfindungsgemäßen Bänderspannvorrichtung.

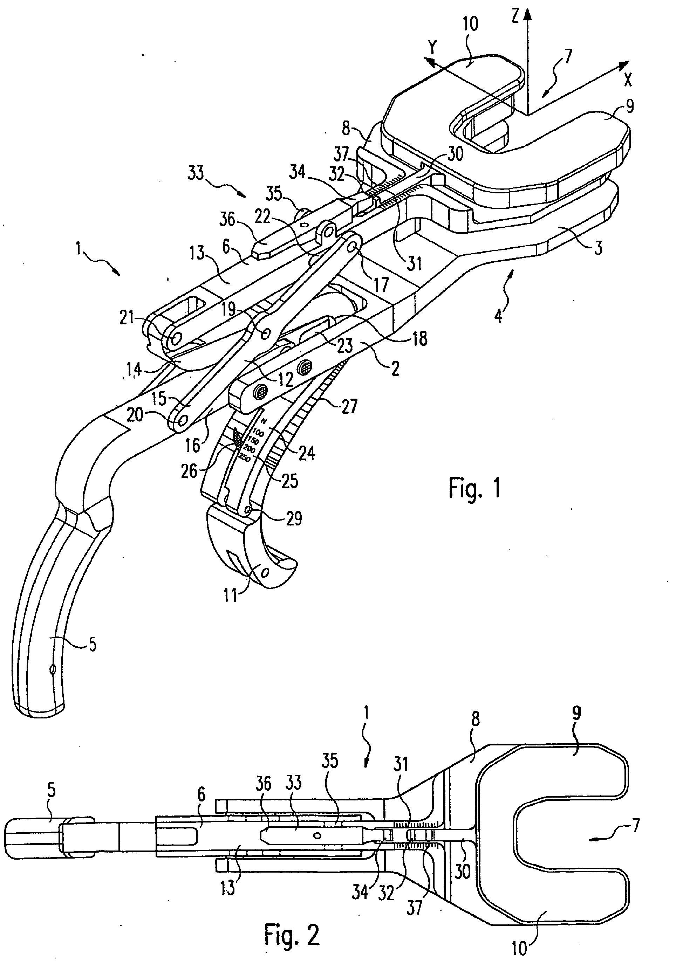

Fig. 1 zeigt in einer schematischen, perspektivischen Gesamtdarstellung ein Ausführungsbeispiel einer erfindungsgemäß ausgestalteten Bänderspannvorrichtung 1 für ein Kniegelenk, bei der eine Verschiebbarkeit der Pratzen zueinander in anterio-posteriorer Richtung verwirklicht ist.

Die Bänderspannvorrichtung 1 umfaßt einen Grundkörper 2, welcher zur sicheren Einleitung der Spreizkraft in die Tibia über eine erste Pratze 3 mit einer in Bezug auf den Kniegelenksspalt distalen Auflagefläche 4 verfügt, welche im Fall des Kniegelenks an der Tibia anliegt. Der ersten Pratze 3 gegenüberliegend ist entsprechend am Grundkörper 2 ein Handgriff 5 angebracht, welcher ein einhändiges Halten und Spannen der Bänderspannvorrichtung 1 ermöglicht.

Ebenfalls entsprechend zur Anordnung der ersten Pratze 3 und oberhalb dieser liegend umfaßt die Bänderspannvorrichtung 1 einen Spannhebel 6, an welchem eine zweite Pratze 7 angeordnet ist. Die zweite Pratze 7 ist dabei erfindungsgemäß zweiteilig ausgebildet. Ein erster Teil 8 ist mit dem Spannhebel 6 verbunden, ein zweiter Teil 9 ist in Bezug auf den Kniegelenksspalt proximal zum ersten Teil 8 verschieblich zu diesem angeordnet. Der zweite Teil 9 weist eine proximale Auflagefläche 10 auf, welche sich auf dem gegenüberliegenden Anteil des zu behandelnden Gelenkes, im Fall des Kniegelenks dem Femur, abstützt. Die Spreizwirkung wird durch Betätigen des Handgriffs 5 zusammen mit einem Bedienungshebel 11 erzeugt.

Eine Parallelverschiebevorrichtung 12 gestattet bezüglich der Auflageflächen 4 und 10 eine Parallelverschiebung der zweiten Pratze 7 mit der Auflagefläche 10 gegenüber der ersten Pratze 3 mit der Auflagefläche 4. Die zweite Pratze 7 steht dabei in Wirkverbindung mit dem Spannhebel 6. Die Parallelverschiebevorrichtung 12 ist im Ausführungsbeispiel als Viergelenk in Form sich kreuzender Stäbe ausgeführt und umfaßt vier Hebel 13, 14, 15, 16, wobei ein spannhebelseitiger Hebel 13 und ein grundkörperseitiger Hebel 16 parallel angeordnet sind, während sich die Hebel 14 und 15 kreuzen. Die vier Hebel 13, 14, 15, 16 sind mittels fünf Achsen 17, 18, 19, 20, 21 miteinander verbunden. Zwei der Achsen 17, 18 sind in den parallelen Hebeln 13, 16 in parallel zu den Auflageflächen 4, 10 verlaufenden Nuten 22, 23 verschiebbar gelagert.

Diese Ausgestaltung der Parallelverschiebevorrichtung 12 gestattet, daß der spannhebelseitige Hebel 13 und der grundkörperseitige Hebel 16 parallel zueinander bzw. auseinander bewegbar sind. Die Längen der Hebel 13, 14, 15, 16 sind so gewählt, daß bei einer beliebigen Spannweite X zwischen der Auflagefläche 4 an der ersten Pratze 3 und der Auflagefläche 10 an der zweiten Pratze 7, welche z. B. zwischen 5 mm und 40 mm liegen kann, ein bestimmtes Umsetzungsverhältnis zwischen der manuell an dem Handgriff 5 und dem Bedienungshebel 11 aufgebrachten Spannkraft und der auf die an das Gelenk angrenzenden Knochen ausgeübten Distraktionskraft herrscht.

Die Größe der Spreizkraft ist an einer Kraftanzeige 24 mit einer Skala 25 und einem beweglichen Anzeigehebel 26 ablesbar. Der Anzeigehebel 26 wird durch die longitudinale Biegung des durch eine manuell aufgebrachte Spannkraft biegbaren Bedienungshebelteils 27 gegenüber dem anderen gabelartig angeordneten und nicht durch diese Spannkraft beaufschlagten Anzeigehebel 26 bewegt. Werden mittels der Spannkraft der Anzeigehebel 26 und das Bedienungshebelteil 27 relativ zueinander bewegt, dreht sich der Anzeigehebel 26 um eine Drehachse 29, wodurch auf der Skala 25 durch den Anzeigehebel 26 die manuell aufgebrachte Spannkraft angezeigt wird.

Weiterhin kann zwischen dem Handgriff 5 und dem Bedienungshebel 11 eine in Fig. 1 nicht weiter dargestellte Arretierungsvorrichtung vorgesehen sein, welche die Arretierung der Bänderspannvorrichtung 1 in einer bestimmten Position ermöglicht.

Wie bereits weiter oben erwähnt, ist die zweite, proximale Pratze 7 zweiteilig ausgebildet. Der erste, distale Teil 8 ist dabei mit dem Spannhebel 6 verbunden oder einstückig ausgebildet. Auf dem ersten Teil 8 ist der zweite, proximale Teil 9 angeordnet, welcher beispielsweise hufeisenförmig ausgebildet sein kann, um der Form der Femurkondylen, welche sich darauf abstützen, Rechnung zu tragen.

Der proximale Teil 9 weist einen Dorn 30 auf, der sich in anterio-posteriorer Richtung in eine Führung 31, welche an dem Hebel 13 ausgebildet ist, erstreckt. Der Dorn weist Rasten 32 auf, in welche eine Arretierungsvorrichtung 33, die im Ausführungsbeispiel ähnlich einem Kipphebel oder einer Wippe ausgebildet ist, mit einem entsprechend geformten Fortsatz 34 eingreift. Die Arretierungsvorrichtung 33 pivotiert dabei um eine Achse 35, welche auf dem Hebel 13 angeordnet ist. Ein freies Ende 36 der Arretierungsvorrichtung 33 dient der Betätigung der Arretierungsvorrichtung 33.

Fig. 2 zeigt eine schematische Aufsicht auf das in Fig. 1 dargestellte Ausführungsbeispiel einer erfindungsgemäßen Bänderspannvorrichtung 1. Gleiche Bauteile sind dabei mit übereinstimmenden Bezugszeichen versehen.

Wie bereits in Fig. 1 erläutert, weist die erfindungsgemäß ausgestaltete Bänderspannvorrichtung 1 eine gegenüber der distalen Pratze 3 zumindest teilweise verschiebbare proximale Pratze 7 auf, an deren Anlagefläche 10 sich bei einem Kniegelenkseingriff die Femurkondylen abstützen.

In Fig. 2 ist der proximale Teil 9 der zweiten, proximalen Pratze 7 mit der Anlagefläche 10 in entrastetem Zustand dargestellt. Die an dem Dorn 30 erkennbaren Rasten 32 befinden sich nicht mehr in Eingriff mit dem Fortsatz 34 der Arretierungsvorrichtung 33.

Die Funktionsweise der erfindungsgemäß ausgestalteten Bänderspannvorrichtung ist dabei folgendermaßen:

Wird zur Vorbereitung einer Kniegelenksimplantation eine Vorrichtung zum Spannen der Ligamente in den Kniegelenksspalt eingeführt und um einen Betrag X aufgespreizt, können, bedingt durch die Überlagerung der Spannungen in Ligamenten und anderen Weichteilen, schräg, d. h. nicht in cranio-caudaler oder anterio-posteriorer Richtung, wirkende Kräfte zwischen der Spannvorrichtung und den an dieser anliegenden Knochen kommen. Durch diese Spannungen, die prinzipiell in beliebiger Richtung wirken können, kann dies einerseits infolge daraus resultierender Reibkräfte, zu einer Verfälschung der zu quantifizierenden cranio-caudalen Kraft und andererseits zu Störungen des Operationsablaufs führen, wenn beispielsweise die Knochen auf der Oberfläche der Vorspannvorrichtung abgleiten oder sich Spannungen dadurch unkontrolliert oder ruckartig abbauen.

Um dies zu verhindern, besitzt die erfindungsgemäß ausgestaltete Bänderspannvorrichtung 1 eine in anterio-posteriorer Richtung verschieblich angeordnete Pratze 7, welche relativ zu der anderen Pratze 3 beweglich ist. Die verschiebliche Pratze 7 ist mittels der schon beschriebenen Arretierungsvorrichtung 33 während des Einführens und Aufspreizens der Bänderspannvorrichtung 1 arretiert. Ist die gewünschte Spreizkraft erreicht, kann der Operateur vorzugsweise mittels des Daumens durch Druck auf das Ende 36 der Arretierungsvorrichtung 33 diese lösen, so daß der Fortsatz 34 nicht mehr in Eingriff mit den Rasten 32 des Dorns 30 steht. Dadurch rutscht der proximale Teil 9 der Pratze 7 unter dem Zug der an der Anlagefläche 10 der Pratze 7 anliegenden Femurkondylen so lange in posteriorer Richtung, bis das Kniegelenk in dieser Richtung spannungsfrei ist. Dadurch kann sich während der Operation kein ruckartiger Spannungsabbau ereignen. Zudem wird die Reibungskraft auf die Knochenhaut vermindert und diese dadurch geschont.

Eine an der Führung 31 angebrachte Skala 37 ermöglicht eine Quantifizierung der anterio-posterioren Verschiebung. Dies ist beispielsweise von Nutzen, um die Reaktion des cranio-caudalen Aufspannens und damit den Einfluß des hinteren Kreuzbandes und anderer Weichteilsysteme auf die relative Verschiebung des Femurs gegenüber der Tibia zu erfassen und zudem einzelne Schritte der Weichteilablösung (Release) zu kontrollieren. Zudem dient diese Quantifizierung zur Objektivierung von Erfahrungen durchgeführter Operationen, wodurch die daraus gesammelten Erkenntnisse zwecks erhöhter Reproduzierbarkeit in zukünftige Operationen einfließen können.

Es ist vorteilhaft, eine entsprechende Einrichtung auch für die anderen translatorischen und rotatorischen Freiheitsgrade vorzusehen. Beispielsweise ist es problemlos möglich, eine lateral-mediale Bewegung durch eine weitere Führung des zweiten Teils 9 der proximalen Pratze 7 zu ermöglichen. Auch rotatorische Freiheitsgrade, welche Drehungen um verschiedene Achsen ermöglichen, sind denkbar. Kugelgelenkverbindungen zwischen den Pratzen 3 und 7 und der Bänderspannvorrichtung 1 würden beispielsweise Verkippungen der Pratzen 3 und 7 gegeneinander ermöglichen und sich zwanglos in das beschriebene Ausführungsbeispiel einfügen lassen. Vorteile solcher Erweiterungen sind insbesondere die problemlosen Anpassungen an die individuellen anatomischen Randbedingungen eines beliebigen (Knie-) Gelenks und das zwanglose Aufspreizen der Vorrichtung zur Quantifizierung der realen cranio-caudalen Reaktionskräfte.

## Patentansprüche

1. Bänderspannvorrichtung (1) zur Aktivierung des Band- und/oder Kapselapparates während der Implantierung eines Gelenksimplantats mit einem Grundkörper (2), welcher eine erste Pratze (3) mit einer distalen Anlagefläche (4), welche auf einem ersten Knochen aufliegt, und eine zweite Pratze (7), die mit einer proximalen Auflagefläche (10) an einem zweiten Knochen anliegt, aufweist,
**dadurch gekennzeichnet,**
**daß** die zweite Pratze (7) in anterio-posteriorer Richtung und/oder medial-lateraler Richtung parallel zur ersten Pratze (3) verschieblich ist.

2. Bänderspannvorrichtung nach Anspruch 1,
**dadurch gekennzeichnet,**
**daß** die zweite Pratze (3) einen distalen Teil (8) und einen proximalen Teil (9) umfaßt.

3. Bänderspannvorrichtung nach Anspruch 2,
**dadurch gekennzeichnet,**
**daß** der distale Teil (8) gegenüber dem proximalen Teil (9) verschieblich ist.

4. Bänderspannvorrichtung nach Anspruch 3,
**dadurch gekennzeichnet,**
**daß** der distale Teil (8) der zweiten Pratze (7) eine Führung (31) aufweist.

5. Bänderspannvorrichtung nach Anspruch 4,
**dadurch gekennzeichnet,**
**daß** ein an dem proximalen Teil (9) der zweiten Pratze (7) ausgebildeter Dorn (30) in der Führung (31) geführt ist.

6. Bänderspannvorrichtung nach Anspruch 5,
**dadurch gekennzeichnet,**
**daß** die Führung (31) eine Skala (37) aufweist.

7. Bänderspannvorrichtung nach Anspruch 5 oder 6,
**dadurch gekennzeichnet,**
**daß** der Dorn (30) Rasten (32) aufweist.

8. Bänderspannvorrichtung nach Anspruch 7,
**dadurch gekennzeichnet,**
**daß** an der zweiten Pratze (7) eine Arretierungsvorrichtung (33) vorgesehen ist.

9. Bänderspannvorrichtung nach Anspruch 8,
**dadurch gekennzeichnet,**
**daß** die Arretierungsvorrichtung (33) beweglich in die Rasten (32) eingreift.

10. Bänderspannvorrichtung nach Anspruch 9,
**dadurch gekennzeichnet,**
**daß** die Arretierungsvorrichtung (33) als um eine Achse (35) pivotierender Kipp- oder Wipphebel ausgebildet ist.

11. Bänderspannvorrichtung nach einem der Ansprüche 8 bis 10,
**dadurch gekennzeichnet,**
**daß** der proximale Teil (9) der zweiten Pratze (7) gegenüber dem distalen Teil (8) der zweiten Pratze (7) durch Betätigung Arretierungsvorrichtung (33) freigebbar ist.

12. Bänderspannvorrichtung nach einem der Ansprüche 1 bis 11,
**dadurch gekennzeichnet,**
**daß** die erste Pratze (3) und die zweite Pratze (7) mittels einer Parallelverschiebevorrichtung (12) in cranio-caudaler Richtung parallel zueinander verschiebbar sind.

13. Bänderspannvorrichtung nach Anspruch 12,
**dadurch gekennzeichnet,**
**daß** die Bänderspannvorrichtung (1) eine Kraftanzeige (24) für die durch die Parallelverschiebevorrichtung (12) in cranio-caudaler Richtung applizierte Kraft aufweist.

14. Bänderspannvorrichtung nach Anspruch 13,
**dadurch gekennzeichnet,**
**daß** die anterio-posteriore und/oder medial-laterale Verschiebung der ersten Pratze (3) und der zweiten Pratze (7) zueinander unabhängig von der cranio-caudalen Verschiebung der ersten Pratze (3) und der zweiten Pratze (7) zueinander durchführbar ist.

15. Bänderspannvorrichtung nach einem der Ansprüche 1 bis 14,
**dadurch gekennzeichnet,**
**daß** die zweite Pratze (7) so angeordnet ist, daß eine Rotation der zweiten Pratze (7) in einer varus-valgus Richtung, in intern-externen Richtung und in Flexions-Extensionsrichtung relativ zu der ersten Pratze (3) durchführbar ist.

16. Bänderspannvorrichtung nach Anspruch 15,
**dadurch gekennzeichnet,**
**daß** die Rotationen in varus-valgus Richtung, in internexterner Richtung und in Flexions-Extensionsrichtung unabhängig voneinander durchführbar sind.

## Claims

1. A ligament-tensioning device (1) for activating the ligament and/or capsule apparatus during implantation of a joint implant, having a base member (2) comprising a first claw (3) with a distal bearing surface (4), which bears on a first bone, and a second claw (7), which bears with a proximal bearing surface (10) on a second bone,
**characterised in that**
the second claw (7) may be displaced in an anteroposterior direction and/or mediolateral direction parallel to the first claw (3).

2. A ligament-tensioning device according to claim 1,
**characterised in that**
the second claw (3) comprises a distal part (8) and a proximal part (9).

3. A ligament-tensioning device according to claim 2,
**characterised in that**
the distal part (8) is displaceable relative to the proximal part (9).

4. A ligament-tensioning device according to claim 3,
**characterised in that**
the distal part (8) of the second claw (7) comprises a guide (31).

5. A ligament-tensioning device according to claim 4,
**characterised in that**
a projection (30) formed on the proximal part (9) of the second claw (7) is guided in the guide (31).

6. A ligament-tensioning device according to claim 5,
**characterised in that**
the guide (31) comprises a scale (37).

7. A ligament-tensioning device according to claim 5 or claim 6,
**characterised in that**
the projection (30) comprises catches (32).

8. A ligament-tensioning device according to claim 7,
**characterised in that**
a locking device (33) is provided on the second claw (7).

9. A ligament-tensioning device according to claim 8,
**characterised in that**
the locking device (33) engages movably in the catches (32).

10. A ligament-tensioning device according to claim 9,
**characterised in that**
the locking device (33) takes the form of a tilting or rocking arm pivoting about an axis (35).

11. A ligament-tensioning device according to any one of claims 8 to 10,
**characterised in that**
the proximal part (9) of the second claw (7) may be released relative to the distal part (8) of the second claw (7) by actuation of the locking device (33).

12. A ligament-tensioning device according to any one of claims 1 to 11,
**characterised in that**
the first claw (3) and the second claw (7) may be displaced parallel to one another in the craniocaudal direction by means of a parallel displacement device (12).

13. A ligament-tensioning device according to claim 12,
**characterised in that**
the ligament-tensioning device (1) comprises a force display (24) for the force applied in a craniocaudal direction by the parallel displacement device (12).

14. A ligament-tensioning device according to claim 13,
**characterised in that**
the anteroposterior and/or mediolateral displacement of the first claw (3) and the second claw (7) relative to one another may be effected independently of the craniocaudal displacement of the first claw (3) and the second claw (7) relative to one another.

15. A ligament-tensioning device according to any one of claims 1 to 14,
**characterised in that**
the second claw (7) is arranged in such a way that rotation of the second claw (7) relative to the first claw (3) may be effected in a varus-valgus direction, in an internal-external direction and in the flexion-extension direction.

16. A ligament-tensioning device according to claim 15,
**characterised in that**
the rotations in the varus-valgus direction, in the internal-external direction and in the flexion-extension direction may be effected independently of one another.

## Revendications

1. Dispositif tendeur de ligaments (1) pour l'activation de l'appareil ligamentaire et/ou capsulaire pendant l'implantation d'un implant articulaire avec un corps de base (2), lequel corps de base présente une première patte (3) avec une surface d'appui distale qui repose sur un premier os, et une seconde patte (7) qui repose par une surface d'appui proximale (10) sur un second os,
**caractérisé**
**en ce que** la seconde patte (7) est déplaçable dans la direction antéropostérieure et/ou dans la direction médiane-latérale parallèlement à la première patte (3).

2. Dispositif tendeur de ligaments selon la revendication 1,
**caractérisé**
**en ce que** la seconde patte (3) comprend une partie distale (8) et une partie proximale (9).

3. Dispositif tendeur de ligaments selon la revendication 2,
**caractérisé**
**en ce que** la partie distale (8) est déplaçable par rapport à la partie proximale (9).

4. Dispositif tendeur de ligaments selon la revendication 3,
**caractérisé**
**en ce que** la partie distale (8) de la seconde patte (7) comprend une glissière (31).

5. Dispositif tendeur de ligaments selon la revendication 4,
**caractérisé**
**en ce qu'**un mandrin (30) formé sur la partie proximale (9) de la seconde patte (7) est guidé dans la glissière (31).

6. Dispositif tendeur de ligaments selon la revendication 5,
**caractérisé**
**en ce que** le glissière (31) présente une échelle graduée (37).

7. Dispositif tendeur de ligaments selon la revendication 5 ou 6,
**caractérisé**
**en ce que** le mandrin (30) présente des encoches (32).

8. Dispositif tendeur de ligaments selon la revendication 7,
**caractérisé**
**en ce qu'**un dispositif de positionnement (33) sur la seconde patte (7) est prévu.

9. Dispositif tendeur de ligaments selon la revendication 8,
**caractérisé**
**en ce que** le dispositif de positionnement (33) s'engage de façon mobile dans les encoches (32).

10. Dispositif tendeur de ligaments selon la revendication 9,
**caractérisé**
**en ce que** le dispositif de positionnement (33) est configuré comme un levier de basculement ou de pivotement autour d'un axe (35).

11. Dispositif tendeur de ligaments selon l'une des revendications 8 à 10,
**caractérisé**
**en ce que** la partie proximale (9) de la seconde patte (7) est libérable par rapport à la partie distale (8) de la seconde patte (7) par actionnement du dispositif de positionnement (33).

12. Dispositif tendeur de ligaments selon l'une des revendications 1 à 11,
**caractérisé**
**en ce que** la première patte (3) et la seconde patte (7) sont déplaçables parallèlement entre elles au moyen d'un dispositif de déplacement parallèle (12) dans la direction cranio-caudale.

13. Dispositif tendeur de ligaments selon la revendication 12,
**caractérisé**
**en ce que** le dispositif tendeur de ligaments (1) présente un indicateur de force (24) pour la force appliquée par le dispositif de déplacement parallèle (12) dans la direction cranio-caudale.

14. Dispositif tendeur de ligaments selon la revendication 13,
**caractérisé**
**en ce que** le déplacement antéropostérieur et/ou médian-latéral de la première patte (3) et de la seconde patte (7) peut être effectué indépendamment l'un de l'autre à partir du déplacement cranio-caudal de la première patte (3) et de la seconde patte (7).

15. Dispositif tendeur de ligaments selon l'une des revendications 1 à 14,
**caractérisé**
**en ce que** la seconde patte (7) est disposée de sorte qu'une rotation de la seconde patte (7) dans une direction varus-valgus peut être effectuée dans la direction interne-externe et dans la direction de flexion-extension par rapport à la première patte (3).

16. Dispositif tendeur de ligaments selon la revendication 15,
**caractérisé**
**en ce que** les rotations dans la direction varus-valgus peuvent être effectuées dans la direction interne-externe et dans la direction de flexion-extension indépendamment l'une de l'autre.
